# EUROPEAN PATENT SPECIFICATION

(11) **EP 3 760 609 B1**
(45) Date of publication and mention of the grant of the patent: **05.10.2022**
(21) Application number: 20772769.4
(22) Date of filing: 28.02.2020
(51) Int. Cl.: C07C 209/48, C07C 209/84, C07C 211/27, B01J 23/75, B01J 23/28, B01J 23/00, B01J 27/199, B01J 37/00, B01J 37/08, B01J 23/755, B01J 25/00

(54) **METHOD FOR PRODUCING XYLYLENEDIAMINE**
VERFAHREN ZUR HERSTELLUNG VON XYLYLENDIAMIN
PROCÉDÉ DE PRODUCTION DE XYLYLÈNEDIAMINE

(30) Priority: 20.03.2019 JP 2019053082
(43) Date of publication of application: 06.01.2021
(73) Proprietor: MITSUBISHI GAS CHEMICAL COMPANY, INC., Chiyoda-ku Tokyo 100-8324 (JP)
(72) Inventor: IBI, Yukiya, Kurashiki-shi, Okayama 712-8525 (JP); KUMANO, Tatsuyuki, Kurashiki-shi, Okayama 712-8525 (JP)
(74) Representative: Hoffmann Eitle
(86) International application number: PCT/JP2020/008407
(87) International publication number: WO 2020/189227

(56) References cited:
- EP-A1- 1 873 137
- WO-A1-2010/026920
- WO-A1-2012/105498
- JP-A- 2007 332 135
- JP-A- 2007 332 135
- JP-A- 2007 533 614
- JP-A- 2010 065 031
- JP-A- 2010 168 374
- US-A1- 2012 172 616

## Description

### Technical Field

The present invention relates to a method for producing xylylenediamine by hydrogenating dicyanobenzene.

### Background Art

Xylylenediamine is a compound useful as a raw material of a polyamide resin, a curing agent, and the like, and an intermediate material of an isocyanate resin and the like. A method for hydrogenating dicyanobenzene has been examined as the method for producing xylylenediamine.

For example, Patent Document 1 describes that a nickel-copper-molybdenum-based catalyst is used to catalytically reducing dicyanobenzene, which is brought into contact with hydrogen in liquid phase. Patent Document 2 describes that meta-xylylenediamine can be produced by subjecting isophthalonitrile to batch hydrogenation reaction by an autoclave in the presence of a hydroxide or an alcoholate of an alkali or alkali earth metal and a Raney nickel or Raney Cobalt catalyst in a mixed solvent of a lower alcohol and a cyclic hydrocarbon.

In recent years, methods for obtaining xylylenediamine with higher purity have also been examined.

For example, Patent Document 3 describes a method for alkali-treating a crude xylylenediamine, and Patent Document 4 describes a method for contacting a crude xylylenediamine with a catalyst containing an iron oxide or an oxide of iron and chromium in the presence of water.

Patent Document 5 describes that highly pure xylylenediamine can be produced with good yield by hydrogenating dicyanobenzene in two stages in the presence of a solvent.

Patent Document 6 describes a production method in which after a first contact hydrogenation reaction of dicyanobenzene is performed using liquid ammonia or a mixed solvent including liquid ammonia and an organic solvent, liquid ammonia is removed to form a reaction product and the resulting reaction product is subjected to a second contact hydrogenation reaction under mild conditions.

Patent Document 7 describes a method of producing xylylenediamine by a two-stage hydrogenation of a starting phthalonitrile in a solvent. The main steps of the method are a hydrogenation step 1 and a hydrogenation step 2. In the hydrogenation step 1, a solution of the starting phthalonitrile in the solvent containing liquid ammonia is fed to an inlet of a first reaction zone and the hydrogenation is carried out in the first reaction zone in the presence of a heterogeneous catalyst, to hydrogenate nitrile groups in the starting phthalonitrile to aminomethyl groups. Apart of the hydrogenation product solution from the first reaction zone is circulated to the inlet of the first reaction zone and the rest is introduced into the hydrogenation step 2 where further undergoes the hydrogenation.

Patent Document 8 relates to a process comprising at least the steps (A) chemical reaction of at least one organic compound in the presence of at least one heterogeneous catalyst in a reaction mixture and (B) removal of the at least one heterogeneous catalyst by means of a magnetic filter, and also the use of a magnetic filter for separating off catalyst particles in a process for the hydrogenation of at least one organic compound.

### Citation List

### Patent Documents

Patent Document 1: JP S49-133339 A
Patent Document 2: JP S54-41804 A
Patent Document 3: JP S45-14777 B
Patent Document 4: JP S57-27098 B
Patent Document 5: JP 2004-292435 A
Patent Document 6: JP 2007-332135 A
Patent Document 7: EP 1 873 137 A
Patent Document 8: US 2012/172616 A1

### Summary of Invention

### Technical Problem

Recently, among the applications of xylylenediamine, highly pure xylylenediamine having a low content of cyanobenzylamine, which is a reaction intermediate, is particularly desired for use as an intermediate raw material for isocyanate with excellent quality. However, the hydrogenation reaction using dicyanobenzene as a raw material proceeds in a serial reaction from dicyanobenzene to cyanobenzylamine and from cyanobenzylamine to xylylenediamine, and thus cyanobenzylamine is likely to remain in the resulting xylylenediamine. In addition, the difference between the boiling points of cyanobenzylamine and corresponding xylylenediamine is generally small, and thus it is difficult to separate cyanobenzylamine from xylylenediamine by distillation.

The production method described in Patent Document 6 is a useful method for producing highly pure xylylenediamine with a low cyanobenzylamine content. However, the problem has been found that in this production method, when the reaction is performed for an extended period of time, a differential pressure occurs in the reactor in which the second contact hydrogenation reaction is performed, whereby it is difficult to continuously produce xylylenediamine with a low cyanobenzylamine content. Therefore, from an industrial perspective, there has been a demand for a method that can stably produce a highly pure xylylenediamine over a longer period of time.

An object of the present invention is to provide a method that can stably produce a highly pure xylylenediamine with a low cyanobenzylamine content over an extended period of time.

### Solution to Problem

As a result of diligent studies, the present inventors have found that in a method of producing xylylenediamine by performing a two-stage hydrogenation reaction using dicyanobenzene as a raw material, it is possible to stably produce a highly pure xylylenediamine over an extended period of time by incorporating a solid-liquid separation step.

That is, the present invention provides a method for producing xylylenediamine, as specified in the appended claims, including the following sequential steps:
(1) performing a first hydrogenation including hydrogenating a mixed solution including dicyanobenzene and a solvent containing liquid ammonia in a fixed-bed reactor to form a reaction product (A);
(2) performing ammonia separation including separating and removing the liquid ammonia included in the reaction product (A) to form a reaction product (B);
(3) performing solid-liquid separation including subjecting the reaction product (B) to solid-liquid separation and removing a solid component to form a reaction product (C); and
(4) performing a second hydrogenation including hydrogenating the reaction product (C) in a fixed-bed reactor.

### Advantageous Effects of Invention

According to the production method of the present invention, highly pure xylylenediamine having a low cyanobenzylamine content is stably produced over an extended period of time.

### Brief Description of Drawings

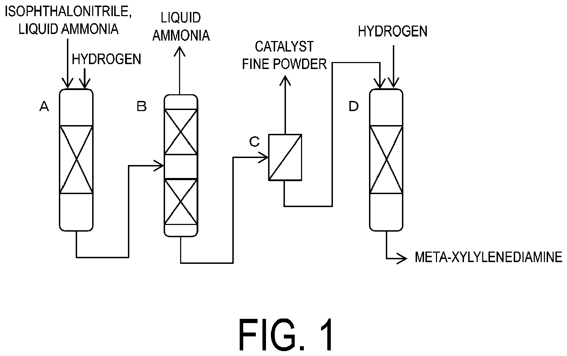

FIG. 1 is a process flow sheet illustrating the present invention, that is, steps of hydrogenating dicyanobenzene to produce xylylenediamine. In FIG. 1, A denotes a first hydrogenation reactor, B denotes an ammonia separation distillation column, C denotes a solid-liquid separation apparatus, and D denotes a second hydrogenation reactor.

### Description of Embodiments

The method for producing xylylenediamine of the present invention includes the following sequential steps:
(1) performing a first hydrogenation including hydrogenating a mixed solution including dicyanobenzene and a solvent containing liquid ammonia in a fixed-bed reactor to form a reaction product (A);
(2) performing ammonia separation including separating and removing the liquid ammonia included in the reaction product (A) to form a reaction product (B);
(3) performing solid-liquid separation including subjecting the reaction product (B) to solid-liquid separation and removing a solid component to form a reaction product (C); and
(4) performing a second hydrogenation including hydrogenating the reaction product (C) in a fixed-bed reactor.

The reason why highly pure xylylenediamine can be stably produced for an extended period of time by the production method according to an embodiment of the present invention is not clear, but the following can be considered as one reason.

It can be considered that thorough removal of the catalyst fine powder flowing out of the reactor used in the first hydrogenation reaction of the two-stage hydrogenation reaction before the reactor used for the second hydrogenation reaction can prevent fine powder of the catalyst from depositing in the reactor used in the second hydrogenation reaction, and thus effectively suppress deterioration of the xylylenediamine caused by the deposited catalyst fine powder and aggregation of the catalyst promoted. Therefore, it is possible to stably produce highly pure xylylenediamine for an extended period of time without changing the conditions of the second hydrogenation reaction.

Each of the steps of the production method according to an embodiment of the present invention will be described in detail below.

### [Method for producing xylylenediamine]

### (1) First hydrogenation

The present step is hydrogenating a mixed solution including dicyanobenzene and a solvent containing liquid ammonia in a fixed-bed reactor to form a reaction product (A).

### (Dicyanobenzene)

Dicyanobenzene used in the present step may be formed by any method, but it is industrially preferable to be formed by ammoxidation reaction of xylene.

The ammoxidation reaction can be performed by a known method, and can be performed by feeding and reacting a reaction raw material prepared by mixing a catalyst, xylene, oxygen, and ammonia. The system of the ammoxidation reaction can be either a fluidized bed or a fixed bed. A known catalyst can be used as the catalyst for the ammoxidation, but the catalyst preferably contains vanadium or chromium, and more preferably contains vanadium and chromium.

The amount of ammonia is preferably from 2 to 20 mol per one mol of xylene, and more preferably from 6 to 15 mol. When the amount of ammonia is within the range described above, the yield of dicyanobenzene is good, and the space time yield thereof is also high. Oxygen can be also used as an oxygen-containing gas by diluting oxygen with nitrogen, carbon dioxide, or the like. Air is preferably used as the oxygen-containing gas. The amount of oxygen is preferably 3 mol or more and more preferably 4 to 100 mol per one mol of xylene. When the amount of oxygen is within the range described above, the yield of dicyanobenzene is good, and the space time yield thereof is also high. The reaction temperature is preferably 300 to 500°C and more preferably 330 to 470°C. When the temperature is within the range described above, the conversion rate of xylene is good, the byproduct is suppressed, and dicyanobenzene can be produced at a good yield. The pressure is preferably ordinary pressure (atmospheric pressure) to 300 kPa, and a space velocity of the reaction raw material (Gas Hourly Space Velocity = GHSV) is preferably from 500 to 5000 h⁻¹.

Dicyanobenzene formed in the reaction is collected and used as a raw material in the first hydrogenation.

For the collection of dicyanobenzene, a gaseous ammoxidation reaction product may be cooled to a temperature at which dicyanobenzene is deposited and then be collected, or a gaseous ammoxidation reaction product may be collected with water or a suitable organic solvent. It is preferable to collect the ammoxidation reaction product with one or more organic solvents in which solubility of dicyanobenzene is high and which are inert to dicyanobenzene, and it is more preferable to collect dicyanobenzene with tolunitrile. This dicyanobenzene collection solution may be used as is in the first hydrogenation, but it is preferable to separate some or all of components having a lower boiling point than dicyanobenzene including the organic solvent (low-boiling-point components) by distillation before use in the first hydrogenation. The low boiling point components are removed by distillation, and dicyanobenzene is produced. The organic solvent can be used repeatedly in the collection of the reaction product. In addition, some or all of components having a higher boiling point than that of dicyanobenzene (high-boiling-point components) may be also separated by distillation or extraction.

Dicyanobenzene refers to three isomers, phthalonitrile(1,2-dicyanobenzene), isophthalonitrile(1,3-dicyanobenzene), and terephthalonitrile(1,4-dicyanobenzene), and they can be produced from ortho-xylene, meta-xylene, and para-xylene, which are the corresponding xylenes, according to the ammoxidation method. Using these dicyanobenzenes as raw materials, the corresponding xylylenediamines, that is, orthoxylylenediamine, meta-xylylenediamine, and para-xylylenediamine can be formed. In the production method according to an embodiment of the present invention, preferably, the dicyanobenzene in the first hydrogenation is isophthalonitrile, and the formed xylylenediamine is meta-xylylenediamine.

### (Solvent containing liquid ammonia)

In the present step, dicyanobenzene is dissolved in a solvent containing liquid ammonia, followed by hydrogenation in the presence of a catalyst in a liquid phase.

Examples of the solvent containing liquid ammonia include (i) liquid ammonia, (ii) a mixed solvent of liquid ammonia and an aromatic hydrocarbon, (iii) a mixed solvent of liquid ammonia and xylylenediamine, and (iv) a mixed solvent of liquid ammonia, an aromatic hydrocarbon, and xylylenediamine, and any one selected from these is preferable.

One aromatic hydrocarbon may be used and two or more aromatic hydrocarbons may be used in combination.

From the perspective of increasing the yield in the hydrogenation reaction, the concentration of liquid ammonia in the solvent is preferably higher and specifically, it is more preferably 60 mass% or higher and further preferably 100 mass%.

The amount of the solvent containing liquid ammonia in the present step is preferably 1 to 99 parts by mass, more preferably 3 to 66 parts by mass, and further preferably 5 to 49 parts by mass, per 1 part by mass of dicyanobenzene. When the amount of the solvent is within the range described above, energy required for solvent recovery is low, which is economically advantageous, and the selection rate of xylylenediamine in the hydrogenation reaction is also favorable.

Dicyanobenzene may be dissolved in a solvent containing liquid ammonia by using a mixer such as a static mixer, but from the perspective of preventing a deposited insoluble matter from adhering to the mixer or the like, it is preferable to dissolve dicyanobenzene by mixing dicyanobenzene and the solvent in advance in a dissolution tank. It is more preferable to dissolve dicyanobenzene by feeding molten dicyanobenzene and the solvent into the dissolution tank, and stirring may be performed as necessary.

The pressure and temperature in the dissolution tank are preferably adjusted such that the solution remains in the liquid phase. The pressure in the dissolution tank is preferably from 0.5 to 15 MPa, more preferably from 0.7 to 10 MPa, and further preferably from 1 to 8 MPa. The solution temperature in the dissolution tank is preferably 3 to 140°C, more preferably 5 to 120°C, and further preferably 10 to 100°C.

In a case where an insoluble component is formed in the solution, some or all of the insoluble component may be removed by solid-liquid separation before feeding the solution to the hydrogenation reactor. As the solid-liquid separation, a known method such as filtration, centrifugation, and sedimentation can be used, but filtration is preferable, and filtration by a sintered metal filter and/or strainer is particularly convenient and suitable.

### (Fixed-bed reactor)

The system of the hydrogenation reaction in the present step is a fixed bed, and the hydrogenation reaction is performed using a fixed-bed reactor.

The fixed-bed reaction may be of a batch type or a continuous type, but a continuous type is preferable in order to sufficiently exert the effects of the present invention.

When continuous reaction is performed, a circulating system may be used in which some of the hydrogenation reaction solution formed from an outlet of the hydrogenation reactor is continuously returned to the hydrogenation reactor, or a combination of a circulating system and a one-pass system may be used as described in JP 2008-31155 A. When the reaction is performed continuously, the space velocity of the reaction raw material (Liquid Hourly Space Velocity = LHSV) is preferably from 0.1 to 10 h⁻¹.

When the reaction is performed in batches, the hydrogenation reaction time is preferably from 0.5 to 8 hours.

As the catalyst used in the fixed-bed reactor in the present step, known supported metal catalysts, unsupported metal catalysts, Raney catalysts, sponge catalysts, noble metal catalysts, and the like can be used. Particularly, it is preferable to use a catalyst containing nickel and/or cobalt. The amount of catalyst used may be an amount used for the known liquid phase hydrogenation of dicyanobenzene.

### (Reaction conditions of first hydrogenation)

Hydrogen used in the present step may include impurities such as methane and nitrogen that do not participate in the reaction, but when the impurity concentration is high, the total pressure of the reaction needs to be increased in order to ensure the required hydrogen partial pressure, which is industrially disadvantageous. Thus, the hydrogen concentration is preferably 50 mol% or greater, and more preferably 80 mol% or greater.

In the present step, in order to efficiently produce xylylenediamine, it is essential to increase the degree of progression of the hydrogenation reaction of a nitrile group to an aminomethyl group, and it is preferable to select a reaction condition such that the concentrations of dicyanobenzene and cyanobenzylamine in the liquid formed after the hydrogenation reaction are kept low. Specifically, the amount of cyanobenzylamine relative to xylylenediamine in the solution after the hydrogenation reaction (reaction product (A)) is preferably maintained at 5.0 mass% or less, more preferably 1.0 mass% or less, and further preferably 0.2 mass% or less. In addition, the conversion rate of dicyanobenzene is preferably 99.50% or greater, and more preferably 99.90% or greater, and further preferably 99.95% or greater.

The pressure and temperature of the hydrogenation reaction are preferably adjusted so that the solution remains in the liquid phase. The temperature of the hydrogenation reaction is preferably 20 to 200°C, more preferably 30 to 150°C, and further preferably 40 to 120°C. The hydrogen pressure is preferably 1 to 30 MPa, more preferably 2 to 25 MPa, and further preferably 3 to 20 MPa.

### (2) Ammonia separation

The present step is ammonia separation in which the liquid ammonia included in the reaction product (A) is separated and removed to form a reaction product (B).

The present step includes separating and removing all or part of the liquid ammonia in the solvent including liquid ammonia used in the previous step. In the previous step, when a solvent other than liquid ammonia such as an aromatic hydrocarbon is included, only the liquid ammonia may be separated and removed, and the liquid ammonia and the solvent other than liquid ammonia may be simultaneously separated and removed, but from an industrial perspective, it is preferable to simultaneously separate and remove the liquid ammonia and the solvent other than the liquid ammonia.

The method for separating and removing liquid ammonia is not limited, but separation and removal by distillation is preferable. The distillation is preferably performed under pressurized conditions, and the pressure is preferably from 0.2 to 3 MPa. In addition, the temperature is preferably 50 to 200°C, and more preferably 70 to 180°C.

As a distillation apparatus used in the present step, a known distillation apparatus such as a packed column, a shelf column, and a flash drum can be used, and distillation is performed in batches or continuously.

In addition, the amount of ammonia in the solution containing xylylenediamine formed after distillation of the present step (reaction product (B)) is preferably 1.0 mass% or less. When the amount of ammonia is 1.0 mass% or less, it is possible to prevent an increase in the partial pressure of the solution after distillation, and thus a high-pressure reactor is unnecessary in a subsequent process, which is economically advantageous.

### (3) Solid-liquid separation

The present step is solid-liquid separation including subjecting the reaction product (B) to solid-liquid separation and removing a solid component to form a reaction product (C).

The solid component in the present step is an insoluble matter generated in the step described above, but the main component is considered to be catalyst powder derived from a catalyst used in a fixed-bed reactor, specifically, the main component is considered to be catalyst fine powder having a particle size of 1 to 500 µm.

A known method may be used for the solid-liquid separation, but is preferably adsorption, filtration, or sedimentation, more preferably adsorption or filtration, and further preferably adsorption.

Examples of the adsorption include magnetic adsorption and adsorption by intermolecular forces, and magnetic adsorption is preferable.

It is considered that the use of magnetic adsorption can efficiently remove catalyst fine powder derived from a catalyst having a component including ferromagnetic properties such as nickel and cobalt used in the first hydrogenation, whereby a solution (reaction product (C)) in which little amount of catalyst fine powder remains can be obtained.

The magnetic adsorption is preferably adsorption using a magnet filter.

The magnetic force of the magnet filter is desirably set to be capable of removing the catalyst fine powder, while the size of the magnet filter, the flow rate of the solution, and the like are taken into consideration. Specifically, the magnetic force is preferably from 0.1 to 3 tesla (T) and more preferably from 0.5 to 2 tesla (T).

The filter used for the filtration is not particularly limited, but filtration using a sintered metal filter is preferable.

When a sintered metal filter is used, the filter size of the sintered metal filter is preferably 100 µm or less, and more preferably 50 µm or less, and further preferably 10 µm or less. The sintered metal filter having the filer size of 100 µm or less can efficiently remove the catalyst fine powder flowing out from the first hydrogenation step.

Examples of the sedimentation include static separation and centrifugal separation.

### (4) Second hydrogenation

The present step is a second hydrogenation including hydrogenating the reaction product (C) in a fixed-bed reactor.

The hydrogenation in the present step is preferably performed in a continuous fixed-bed reaction. As the catalyst used in the present step, known supported metal catalysts, unsupported metal catalysts, Raney catalysts, sponge catalysts, noble metal catalysts, and the like can be used. Particularly, catalysts containing nickel and/or cobalt are suitably used.

In a case where the mixed solution includes a solvent other than liquid ammonia such as an aromatic hydrocarbon in the first hydrogenation step, the solvent other than liquid ammonia may be left unremoved in the ammonia separation and used as is in the present step. That is, the reaction product (B) formed in the ammonia separation step (2) may contain a solvent, and the reaction product containing a solvent formed after (3) the solid-liquid separation step may be used as the reaction product (C) in the present step.

The temperature of the hydrogenation reaction is preferably 30 to 150°C, more preferably 40 to 120°C, and further preferably 50 to 100°C. When the temperature is 30°C or higher, decrease in the conversion rate of cyanobenzylamine can be prevented, and when the temperature is 150°C or lower, nuclear hydrogenation and deamination of xylylenediamine can be prevented from proceeding, and thermal alteration of xylylenediamine itself can be prevented. The hydrogen pressure of the hydrogenation reaction is preferably from 0.1 to 10 MPa, more preferably from 0.5 to 8 MPa, and further preferably from 1 to 4 MPa. When the hydrogen pressure is 0.1 MPa or higher, decrease in the conversion rate of cyanobenzylamine can be prevented, and when the hydrogen pressure is 10 MPa or lower, the nuclear hydrogenation and deamination of xylylenediamine can be prevented from proceeding.

When the hydrogenation reaction is performed continuously, the space velocity (LHSV) of the reaction raw material is preferably from 0.1 to 10 h⁻¹, and more preferably from 0.1 to 3.0 h⁻¹. When the space velocity is 0.1 h⁻¹ or greater, the amount that can be handled per unit time increases, which is industrially advantageous. In addition, when the space velocity is 10 h-1 or less, the conversion rate of cyanobenzylamine can be increased.

When the hydrogenation reaction is performed in batches in the present step, the hydrogenation reaction time is preferably from 0.5 to 8 hours.

In the present step, the amount of cyanobenzylamine relative to xylylenediamine in the solution formed after the hydrogenation reaction is preferably maintained at 0.02 mass% or less, more preferably 0.01 mass% or less, further preferably 0.005 mass% or less, and even further preferably 0.001 mass% or less. By appropriately adjusting the reaction conditions such as temperature, hydrogen pressure, reaction time, or LHSV so as to maintain an amount of cyanobenzylamine in the aforementioned range, highly pure xylylenediamine can be formed.

### (5) Purification

The present step is purifying xylylenediamine after the second hydrogenation (4). In an embodiment of the present invention, the method preferably includes the purification (5) from the perspective of purifying the reaction product formed in the second hydrogenation (4) to form a more highly pure xylylenediamine.

The purification method in the present step is preferably a method by distillation, and it is preferable to use a distillation column having a theoretical number of stages of 2 or more, and more preferably a distillation column having a theoretical number of stages of 5 or more.

As the distillation conditions, distillation is preferably performed under a reduced pressure, and the pressure is preferably from 1 to 10 kPa.

That is, the method for producing xylylenediamine according to the present invention is a method including the following sequential steps:
(1) a first hydrogenation including hydrogenating a mixed solution including dicyanobenzene and a solvent containing liquid ammonia in a fixed-bed reactor to form a reaction product (A);
(2) ammonia separation including separating and removing the liquid ammonia included in the reaction product (A) to form a reaction product (B);
(3) solid-liquid separation including subjecting the reaction product (B) to solid-liquid separation and removing a solid component to form a reaction product (C); and
(4) second hydrogenation including hydrogenating the reaction product (C) in a fixed-bed reactor.

### Examples

The present invention will be described more specifically based on Examples described below. The present invention is not limited to these Examples but is defined by the appended claims. In the following Examples, a gas chromatograph was used for composition analysis.

### <Gas chromatography (GC) analysis conditions>

Analysis was performed using a 6890 type GC analyzer available from Agilent Technologies, Inc. equipped with a DB-1 column manufactured by J & W Scientific. The temperature was set to 230°C for an injection port, 295°C for a detector, and from 100°C to 280°C for a column oven (holding for 10 minutes at 100°C, and then raising the temperature to 280°C at a ramp rate of 5°C/minute). Note that samples used in the GC measurement were prepared by diluting each reaction solution with methanol, and then adding diphenylmethane as an internal standard.

### <Synthesis of Isophthalonitrile>

Isophthalonitrile used in the Examples was synthesized as follows.

### (1) Preparation of catalysts for ammoxidation reaction

To 229 g of vanadium pentoxide (available from FUJIFILM Wako Pure Chemical Corporation, guaranteed), 500 mL of water (distilled water) was added, the resulting solution was heated to 80 to 90°C, and 477 g of oxalic acid (available from FUJIFILM Wako Pure Chemical Corporation, guaranteed) was added thereto and dissolved while stirring to prepare a solution of vanadium oxalate. In addition, 400 mL of water was added to 963 g of oxalic acid and heated to 50 to 60°C, and a solution prepared by adding 252 g of chromic anhydride (available from FUJIFILM Wako Pure Chemical Corporation, guaranteed) to 200 mL of water was added thereto while stirring to be dissolved, thereby preparing a solution of chromium oxalate. To the resulting solution of vanadium oxalate, the solution of chromium oxalate was mixed at 50 to 60°C to form a vanadium-chromium solution. To this solution, 41.1 g of phosphomolybdic acid (available from Nippon Inorganic Colour & Chemical Co., Ltd.) H₃(PMo₁₂O₄₀)·20H₂O dissolved in 100 mL of water was added and 4.0 g of potassium acetate (available from FUJIFILM Wako Pure Chemical Corporation, guaranteed) dissolved in 100 mL of water was further added. Then, 2500 g of 20 mass% aqueous silica sol (containing 0.02 wt% Na₂O) was added. To this slurry solution, 78 g of boric acid was added, mixed well, and then heated and concentrated until a liquid volume reached approximately 3800 g. This catalyst solution was spray-dried while maintaining an inlet temperature of 250°C and an outlet temperature of 130°C. After drying for 12 hours in a dryer at 130°C, the product was baked for 0.5 hours at 400°C, and then baked at 550°C for 8 hours under air flow. The catalyst had an atomic ratio of V:Cr:B:Mo:P:Na:K of 1:1:0.5:0.086:0.007:0.009:0.020, and the catalyst concentration was 50 mass%.

### (2) Ammoxidation reaction

An ammoxidation reactor is charged with 6 L of the flow catalyst prepared above, and air, meta-xylene (hereinafter, abbreviated as MX, available from Mitsubishi Gas Chemical Company) and ammonia (available from Mitsubishi Gas Chemical Company) were mixed, then preheated to 350°C and fed to the reactor. The preparation conditions were set such that the MX feed rate was 350 g/h, the molar ratio of ammonia/MX was 11, the molar ratio of oxygen/MX was 5.4, and the space velocity GHSV was 630 h⁻¹. The reaction temperature was set to 420°C and the reaction pressure was set to 0.2 MPa.

### (3) Collection

The gaseous reaction product from the top of the ammoxidation reactor was introduced into an isophthalonitrile absorption column, and isophthalonitrile in the reaction product was absorbed and collected in meta-tolunitrile (available from Mitsubishi Gas Chemical Company). The isophthalonitrile absorption column was made of SUS304, and had a condenser on the upper part thereof, a body part having an inner diameter of 100 mmΦ and a height of 800 mm, a lower part of the body part, which was 450 mm in length, having a structure of a double pipe for allowing steam-heating, and a gas blow inlet being provided at the bottom. Two kg of meta-tolunitrile was placed in the absorption column and the temperature was set to 175°C, and the absorption of the aforementioned ammoxidation reaction product was performed for 2 hours. The solution at the end of the absorption contained 74 mass% of meta-tolunitrile and 25 mass% of isophthalonitrile.

### (4) Low boiling point component separation

The above absorption liquid was fed to the middle stage of a distillation column for low boiling point component separation. For the distillation conditions of the distillation column, a column top pressure was set to 6 kPa, a column top temperature was set to 120°C, a column bottom temperature was set to 183°C, and a residence time at the column bottom was set to 60 minutes. The meta-tolunitrile and other low-boiling point components were distilled off at the column top of the distillation column, and isophthalonitrile was extracted from the column bottom. The purity of the produced isophthalonitrile was 96.4 mass%. The resulting isophthalonitrile was used in the following Examples.

### Example 1

### (1) First hydrogenation

A tubular vertical hydrogenation reactor (in FIG. 1, first hydrogenation reactor A) (made of SUS304, inner diameter of 150 mmcp) was filled with 18 kg of a commercially available supported nickel/diatomaceous earth catalyst (cylindrical shape, diameter of 5 mmcp, height of 5 mm) having a nickel content of 50 mass% and the catalyst was reduced at 200°C under hydrogen gas flow to be activated. After cooling, hydrogen gas was introduced into the reactor under pressure, held at a constant pressure of 8 MPa, and the catalyst layer temperature was maintained at 100°C by heating externally. A mixed solution of isophthalonitrile and liquid ammonia (isophthalonitrile:liquid ammonia = 10:90 (mass ratio)) was continuously fed from the top of the reactor at a rate of 15.0 kg/h while the hydrogen gas was circulated at a flow rate of 1000 NL/h. The amount of reaction intermediate 3-cyanobenzylamine produced increased over time, and the reaction was ended when the amount of 3-cyanobenzylamine relative to meta-xylylenediamine contained in the hydrogenation reaction solution reached 0.2 mass%.

### (2) Ammonia separation

The reaction solution after the first hydrogenation was fed to an ammonia separation distillation column (in FIG. 1, ammonia separation distillation column B), ammonia was distilled and separated at a column bottom temperature of 150°C and 0.5 MPa, and liquid ammonia was recovered from the column top. Finally, the composition of the reaction solution produced from the column bottom was 91.4 mass% meta-xylylenediamine and 0.18 mass% 3-cyanobenzylamine, and no isophthalonitrile was detected. In addition, fine powder of the catalyst used in the first hydrogenation step was found in the produced reaction solution.

### (3) Solid-liquid separation

All of the reaction solution after the ammonia separation was filtered through a magnet filter (in FIG. 1, solid-liquid separation apparatus C) (magnetic force 1 T). In the magnet filter, the catalyst fine powder was selectively removed from the reaction solution. When a total of 610 kg of the reaction solution was filtered, a total of 1.73 g of the catalyst fine powder was recovered by the magnet filter.

### (4) Second hydrogenation

A tubular vertical hydrogenation reactor (in FIG. 1, second hydrogenation reactor D) (made of SUS304, inner diameter of 30 mmcp) was filled with 150 g of a commercially available supported nickel/diatomaceous earth catalyst (cylindrical shape, diameter of 3 mmcp, height of 3 mm) having a nickel content of 50 mass%, and the catalyst was reduced at 200°C under hydrogen gas flow to be activated. After cooling, hydrogen gas was introduced into the reactor under pressure, held at a constant pressure of 2 MPa, and the catalyst layer temperature was maintained at 80°C by heating externally. Thereafter, while circulating hydrogen gas from the upper portion of the reactor at a flow rate of 3 NL/h, the reaction solution after the solid-liquid separation was continuously fed at a rate of 75 g/h.

Increase of the differential pressure between the top and bottom of the catalyst layer was 10 kPa or less even after 300 days from the start of the reaction. The composition of the reaction solution formed after 300 days from the start of the reaction was 91.0 mass% of meta-xylylenediamine and 0.001 mass% or less of 3-cyanobenzylamine.

### (5) Purification

The reaction solution after the second hydrogenation was distilled using a distillation column with a theoretical number of 10 stages under a reduced pressure of 6 kPa to produce meta-xylylenediamine purified to a purity of 99.99%. The 3-cyanobenzylamine content in the produced meta-xylylenediamine was 0.001 mass% or less.

### Example 2

### (1) First hydrogenation

The first hydrogenation was performed in the same manner as in Example 1 except that 18 kg of the supported nickel/diatomaceous earth catalyst, which was a hydrogenation catalyst, was changed to 28.8 kg of a commercially available Raney cobalt catalyst having a cobalt content of 50 mass%, and the reaction was ended at a time when the amount of 3-cyanobenzylamine relative to meta-xylylenediamine in the hydrogenation reaction solution reached 0.2 mass%.

### (2) Ammonia separation

The reaction solution after the first hydrogenation was fed to an ammonia separation distillation column, and ammonia was distilled and separated at a column bottom temperature of 150°C and 0.5 MPa, and liquid ammonia was recovered from the column top. Finally, the composition of the reaction solution produced from the column bottom was 92.8 mass% meta-xylylenediamine and 0.18 mass% 3-cyanobenzylamine, and no isophthalonitrile was detected. In addition, fine powder of the catalyst used in the first hydrogenation was found in the produced reaction solution.

### (3) Solid-liquid separation

All of the reaction solution after the ammonia separation was filtered through a magnet filter (magnetic force 1 T). In the magnet filter, the catalyst fine powder was selectively removed from the reaction solution. When a total of 793 kg of the reaction solution was filtered, a total of 2.72 g of the catalyst fine powder was recovered by the magnet filter.

### (4) Second hydrogenation

The second hydrogenation step was performed in the same manner as in Example 1. Increase of the differential pressure between the top and bottom of the catalyst layer was 10 kPa or less even after 300 days from the start of the reaction. The composition of the reaction solution produced after 300 days from the start of the reaction was 92.4 mass% of meta-xylylenediamine and 0.001 mass% or less of 3-cyanobenzylamine.

### (5) Purification

The reaction solution after the second hydrogenation was distilled using a distillation column with a theoretical number of 10 stages under a reduced pressure of 6 kPa to produce a meta-xylylenediamine purified to a purity of 99.99%. The 3-cyanobenzylamine content in the produced meta-xylylenediamine was 0.001 mass% or less.

### Comparative Example 1

The steps up to the second hydrogenation were performed in the same manner as in Example 1 except that the step of (3) solid-liquid separation was excluded. As a result, the differential pressure between the top and bottom of the catalyst layer gradually increased in the second hydrogenation reactor, the differential pressure reached 300 kPa at the time when 255 days elapsed after the start of the reaction (accumulated fed amount of the reaction solution of 459 kg), and it became impossible to maintain the amount of 3-cyanobenzylamine in the meta-xylylenediamine at 0.001 mass% or less. The composition of the reaction solution produced after 255 days from the start of the reaction was 90.9 mass% of meta-xylylenediamine and 0.011 mass% of 3-cyanobenzylamine.

### Industrial Applicability

According to the present invention, in the hydrogenation of dicyanobenzene to produce xylylenediamine, high quality xylylenediamine can be stably and economically produced for an extended period of time.

### Reference Signs List

A First hydrogenation reactor
B Ammonia separation distillation column
C Solid-liquid separation apparatus
D Second hydrogenation reactor

## Claims

1. A method for producing xylylenediamine , comprising sequential steps of:
(1) performing a first hydrogenation including hydrogenating a mixed solution including dicyanobenzene and a solvent containing liquid ammonia in a fixed-bed reactor to produce a reaction product (A);
(2) performing ammonia separation including separating and removing the liquid ammonia included in the reaction product (A) to produce a reaction product (B);
(3) performing solid-liquid separation including subjecting the reaction product (B) to solid-liquid separation and removing a solid component to produce a reaction product (C); and
(4) performing a second hydrogenation including hydrogenating the reaction product (C) in a fixed-bed reactor, wherein
the steps (1) to (4) are performed in a sequential order.

2. The method for producing xylylenediamine according to claim 1, wherein the solid-liquid separation is adsorption, filtration or sedimentation.

3. The method for producing xylylenediamine according to claim 2, wherein the solid-liquid separation is filtration using a sintered metal filter.

4. The method for producing xylylenediamine according to claim 3, wherein a filter size of the sintered metal filter is 100 µm or less.

5. The method for producing xylylenediamine according to claim 2, wherein the solid-liquid separation is magnetic adsorption.

6. The method for producing xylylenediamine according to claim 5, wherein the magnetic adsorption is adsorption using a magnet filter.

7. The method for producing xylylenediamine according to any one of claims 1 to 6, wherein a catalyst used in the fixed-bed reactor of the performing first hydrogenation (1) is a catalyst containing nickel and/or cobalt.

8. The method for producing xylylenediamine according to any one of claims 1 to 7, wherein a main component of the solid component in the performing solid-liquid separation (3) is catalyst powder derived from a catalyst used in the fixed-bed reactor.

9. The method for producing xylylenediamine according to any one of claims 1 to 8, wherein the solvent containing liquid ammonia is any one selected from: (i) liquid ammonia; (ii) a mixed solvent of liquid ammonia and aromatic hydrocarbon; (iii) a mixed solvent of liquid ammonia and xylylenediamine; and (iv) a mixed solvent of liquid ammonia, aromatic hydrocarbons, and xylylenediamine.

10. The method for producing xylylenediamine according to any one of claims 1 to 9, further comprising (5) performing purification including purifying xylylenediamine after the performing the second hydrogenation (4).

11. The method for producing xylylenediamine according to any one of claims 1 to 10, wherein the dicyanobenzene in the performing the first hydrogenation (1) is isophthalonitrile and a resulting xylylenediamine is meta-xylylenediamine.

12. The method for producing xylylenediamine according to any one of claims 1 to 11, wherein the dicyanobenzene used in the performing the first hydrogenation (1) is produced by an ammoxidation reaction of xylene.

## Patentansprüche

1. Verfahren zur Herstellung von Xylylendiamin, umfassend die folgenden sequentiellen Schritte:
(1) Durchführen einer ersten Hydrierung, einschließlich Hydrieren einer gemischten Lösung, die Dicyanobenzol und ein Lösungsmittel, das flüssiges Ammoniak enthält, in einem Festbettreaktor, um ein Reaktionsprodukt (A) herzustellen;
(2) Durchführen einer Ammoniaktrennung, einschließlich Abtrennen und Entfernen des im Reaktionsprodukt (A) enthaltenen flüssigen Ammoniaks, um ein Reaktionsprodukt (B) herzustellen;
(3) Durchführen einer Fest-Flüssig-Trennung, wobei das Reaktionsprodukt (B) einer Fest-Flüssig-Trennung unterzogen wird und eine feste Komponente entfernt wird, um ein Reaktionsprodukt (C) herzustellen; und
(4) Durchführen einer zweiten Hydrierung, einschließlich Hydrieren des Reaktionsprodukt (C) in einem Festbettreaktor, wobei
die Schritte (1) bis (4) in einer sequentiellen Reihenfolge durchgeführt werden.

2. Verfahren zur Herstellung von Xylylendiamin gemäß Anspruch 1, wobei die Fest-Flüssig-Trennung durch Adsorption, Filtration oder Sedimentation erfolgt.

3. Verfahren zur Herstellung von Xylylendiamin gemäß Anspruch 2, wobei die Fest-Flüssig-Trennung durch Filtration erfolgt, unter Verwendung eines Sintermetallfilters.

4. Verfahren zur Herstellung von Xylylendiamin gemäß Anspruch 3, wobei eine Filtergröße des Sintermetallfilters 100 µm oder weniger beträgt.

5. Verfahren zur Herstellung von Xylylendiamin gemäß Anspruch 2, wobei die Fest-Flüssig-Trennung durch magnetische Adsorption erfolgt.

6. Verfahren zur Herstellung von Xylylendiamin gemäß Anspruch 5, wobei die magnetische Adsorption eine Adsorption unter Verwendung eines Magnetfilters ist.

7. Verfahren zur Herstellung von Xylylendiamin gemäß mindestens einem der Ansprüche 1 bis 6, wobei ein in dem Festbettreaktor der durchzuführenden ersten Hydrierung (1) verwendeter Katalysator ein Katalysator ist, der Nickel und/oder Cobalt enthält.

8. Verfahren zur Herstellung von Xylylendiamin gemäß mindestens einem der Ansprüche 1 bis 7, wobei eine Hauptkomponente der festen Komponente in der durchzuführenden Fest-Flüssig-Trennung (3), ein Katalysatorpulver ist, das von einem in dem Festbettreaktor verwendeten Katalysator stammt.

9. Verfahren zur Herstellung von Xylylendiamin gemäß mindestens einem der Ansprüche 1 bis 8, wobei das Lösungsmittel, das flüssiges Ammoniak enthält, eines ist, ausgewählt aus: (i) flüssigem Ammoniak; (ii) einem gemischten Lösungsmittel aus flüssigem Ammoniak und aromatischem Kohlenwasserstoff; (iii) einem gemischten Lösungsmittel aus flüssigem Ammoniak und Xylylendiamin; und (iv) einem gemischten Lösungsmittel aus flüssigem Ammoniak, aromatischen Kohlenwasserstoffen und Xylylendiamin.

10. Verfahren zur Herstellung von Xylylendiamin gemäß mindestens einem der Ansprüche 1 bis 9, ferner umfassend (5) Durchführen einer Reinigung, einschließlich Reinigen von Xylylendiamin nach der Durchführung der zweiten Hydrierung (4).

11. Verfahren zur Herstellung von Xylylendiamin gemäß mindestens einem der Ansprüche 1 bis 10, wobei das Dicyanobenzol in der durchzuführenden ersten Hydrierung (1) Isophthalonitril ist und ein resultierendes Xylylendiamin meta-Xylylendiamin ist.

12. Verfahren zur Herstellung von Xylylendiamin gemäß mindestens einem der Ansprüche 1 bis 11, wobei das in der durchzuführenden ersten Hydrierung (1) verwendete Dicyanobenzol durch eine Ammoxidationsreaktion von Xylol hergestellt wird.

## Revendications

1. Procédé de production de xylylènediamine, comprenant les étapes séquentielles de :
(1) réalisation d'une première hydrogénation incluant l'hydrogénation d'une solution mixte incluant du dicyanobenzène et un solvant contenant de l'ammoniac liquide dans un réacteur à lit fixe pour produire un produit de réaction (A) ;
(2) réalisation d'une séparation de l'ammoniac incluant la séparation et l'élimination de l'ammoniac liquide inclus dans le produit de réaction (A) pour produire un produit de réaction (B) ;
(3) réalisation d'une séparation solide-liquide incluant la soumission du produit de réaction (B) à une séparation solide-liquide et l'élimination d'un composant solide pour produire un produit de réaction (C) ; et
(4) réalisation d'une seconde hydrogénation incluant l'hydrogénation du produit de réaction (C) dans un réacteur à lit fixe, dans lequel
les étapes (1) à (4) sont réalisées selon un ordre séquentiel.

2. Procédé de production de xylylènediamine selon la revendication 1, dans lequel la séparation solide-liquide est une adsorption, une filtration ou une sédimentation.

3. Procédé de production de xylylènediamine selon la revendication 2, dans lequel la séparation solide-liquide est une filtration à l'aide d'un filtre en métal fritté.

4. Procédé de production de xylylènediamine selon la revendication 3, dans lequel une taille du filtre du filtre en métal fritté est inférieure ou égale à 100 µm.

5. Procédé de production de xylylènediamine selon la revendication 2, dans lequel la séparation solide-liquide est une adsorption magnétique.

6. Procédé de production de xylylènediamine selon la revendication 5, dans lequel l'adsorption magnétique est une adsorption à l'aide d'un filtre magnétique.

7. Procédé de production de xylylènediamine selon l'une quelconque des revendications 1 à 6, dans lequel un catalyseur utilisé dans le réacteur à lit fixe de l'étape de réalisation de la première hydrogénation (1) est un catalyseur contenant du nickel et/ou du cobalt.

8. Procédé de production de xylylènediamine selon l'une quelconque des revendications 1 à 7, dans lequel un composant principal du composant solide dans l'étape de réalisation de la séparation solide-liquide (3) est une poudre de catalyseur provenant d'un catalyseur utilisé dans le réacteur à lit fixe.

9. Procédé de production de xylylènediamine selon l'une quelconque des revendications 1 à 8, dans lequel le solvant contenant de l'ammoniac liquide est l'un quelconque choisi parmi : (i) ammoniac liquide ; (ii) un solvant mixte d'ammoniac liquide et d'hydrocarbure aromatique ; (iii) un solvant mixte d'ammoniac liquide et de xylylènediamine; et (iv) un solvant mixte d'ammoniac liquide, d'hydrocarbures aromatiques et de xylylènediamine.

10. Procédé de production de xylylènediamine selon l'une quelconque des revendications 1 à 9, comprenant en outre (5) la réalisation d'une purification incluant la purification de la xylylènediamine après la réalisation de la seconde hydrogénation (4).

11. Procédé de production de xylylènediamine selon l'une quelconque des revendications 1 à 10, dans lequel le dicyanobenzène dans l'étape de réalisation de la première hydrogénation (1) est un isophthalonitrile et une xylylènediamine obtenue est une méta-xylylènediamine.

12. Procédé de production de xylylènediamine selon l'une quelconque des revendications 1 à 11, dans lequel le dicyanobenzène utilisé dans l'étape de réalisation de la première hydrogénation (1) est produit par une réaction d'ammoxydation du xylène.
